# EUROPEAN PATENT APPLICATION

(11) **EP 3 051 275 A1**
(43) Date of publication of application: **03.08.2016**
(21) Application number: 14849892.6
(22) Date of filing: 02.06.2014
(51) Int. Cl.: G01N 21/64, A61B 10/00, G01N 21/27, G01N 21/65, G01N 33/483

(54) **OPTICAL MEASUREMENT DEVICE**

(30) Priority: 27.09.2013 JP 2013202547
(71) Applicant: FUJIFILM Corporation, Tokyo 106-8620 (JP)
(72) Inventor: MAEDA Kiyohiro, Ashigarakami-gun Kanagawa 258-8538 (JP)
(74) Representative: Klunker . Schmitt-Nilson . Hirsch
(86) International application number: PCT/JP2014/064582
(87) International publication number: WO 2015/045483

(57) **Abstract**

Provided is an optical measurement device which can measure the balance between the oxygen utilization and the oxygen supply in the metabolism of a test subject. An optical measurement device 20 includes a photodetector 22 which measures the amount of a plurality of biological substances contained in a test subject 31 by receiving incidence light R1 and R2 from the test subject 31, and a processing portion 23 which functions as an activation degree calculation portion calculating an activation degree of at least two metabolic pathways among energy metabolism, nucleic acid metabolism, amino acid metabolism, or lipid metabolism based on the amount of the plurality of biological substances detected by the photodetector 22.

## Description

### BACKGROUND OF THE INVENTION

### 1. Field of the Invention

The present invention relates to an optical measurement device which measures the amount of biological substances contained in a test subject by light.

### 2. Description of the Related Art

Knowing the amount of various biological substances contained in a test subject is helpful for diagnosis. For example, a device which simultaneously measures various metabolites of a biological body by mass spectrometry is known (JP5222934B). Furthermore, an optical measurement device which measures the amount of light emitted from nicotinamide adenine dinucleotide (NADH) in a biological body is known. The oxygen saturation of a test subject or the amount of a metabolite such as NADH that is measured by the optical measurement device is used for various types of diagnosis.

### SUMMARY OF THE INVENTION

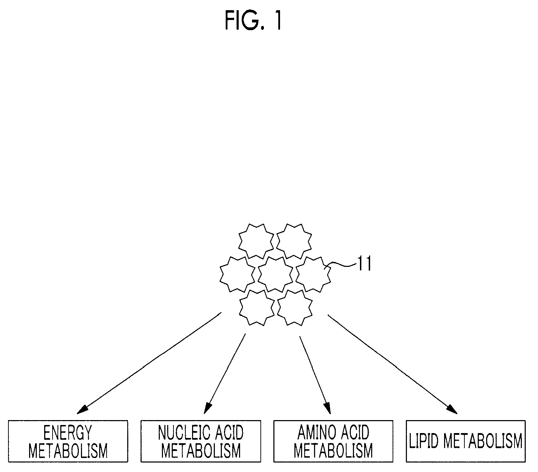

A cancer cell 11 grows more vigorously than a normal cell and thus requires a large amount of energy, nucleic acids, amino acids, and lipids. Therefore, as shown in Fig. 1, a chemical reaction (metabolism) in the cancer cell that produces those requisites is different from the chemical reaction in a normal cell. Furthermore, the activation balance between metabolic pathways of the four requisites varies with the type or state of cancer. Accordingly, by ascertaining the activation balance between the respective metabolic pathways of energy metabolism, nucleic acid metabolism, amino acid metabolism, and lipid metabolism, the information useful for diagnosis is obtained. For example, the activation balance between the four metabolic pathways can be information helpful to determine a degree of malignancy, predict prognosis and drug responsiveness, select a therapeutic method, and the like.

In the four metabolic pathways, hundreds or more biological substances participate in the chemical reaction. According to the device of JP5222934B, by measuring the amount of the hundreds of types of metabolites in a biological body, the activation balance between the four metabolic pathways can be ascertained. However, because the measurement subject has to be dissolved, the mode of the metabolism of the same measurement subject cannot be kept track of over time. As a result, for example, the change after the provision of cancer therapy cannot be kept tracked of. In contrast, in the device of JP2011-053135A, the amount of NADH correlating to the energy metabolism can be ascertained and measured in a nondestructive manner, and hence the mode of the energy metabolism of the same measurement subject can be kept tracked of over time. However, because an activation degree of the energy metabolism is the only thing that can be ascertained, the activation balance between the pathway of the energy metabolism and other metabolic pathways cannot be ascertained. In addition, generally, by the optical measurement disclosed in JP2011-053135A, it is difficult to simultaneously measure the amount of hundreds of types of substances.

An object of the present invention is to provide an optical measurement device which can measure the activation balance between at least two metabolic pathways among four metabolic pathways of energy metabolism, nucleic acid metabolism, amino acid metabolism, or lipid metabolism by means of nondestructive measurement using light.

An optical measurement device of the present invention includes a photodetector which measures the amount of a plurality of biological substances contained in a test subject by receiving light from the test subject and an activation degree calculation portion which calculates an activation degree of each of at least two metabolic pathways among energy metabolism, nucleic acid metabolism, amino acid metabolism, or lipid metabolism based on the amount of the plurality of biological substances detected by the photodetector.

Specifically, the activation degree calculation portion calculates the activation degree by calculating the weighted sum of the amount of the plurality of biological substances. Furthermore, the activation degree calculation portion includes a storage portion which stores in advance coefficients used for calculating the weighted sum.

The storage portion may store in advance a set of coefficients for calculating the weighted sum according to combinations of the biological substances measured by the photodetector. Furthermore, the storage portion may store in advance a set of coefficients according to combinations of metabolic pathways whose activation degree is to be calculated.

The optical measurement device may include a light source which irradiates the test subject with light according to the biological substances whose amount is to be measured by the photodetector. For example, the light source irradiates the test subject with light for measuring light absorption or light scattering performed by the biological substances. Furthermore, the photodetector measures the amount of the biological substances by receiving light which is reflected from or transmitted through the test subject.

The light source may irradiate the test subject with excitation light for exciting a fluorescent substance or a phosphorescent substance in the test subject. In this case, the photodetector measures the amount of the fluorescent substance or the phosphorescent substance by receiving fluorescence or phosphorescence.

The optical measurement device may include a parameter value calculation portion which calculates parameters helpful to determine a degree of malignancy of cancer, predict prognosis and the drug responsiveness, or select a therapeutic method based on the activation degree of the metabolic pathways.

The number of the biological substances whose amount is to be measured by the photodetector is equal to or less than 100 and may be equal to or less than 10.

According to the optical measurement device of the present invention, it is possible to measure the activation balance between at least two metabolic pathways among four metabolic pathways of energy metabolism, nucleic acid metabolism, amino acid metabolism, or lipid metabolism by nondestructive measurement using light.

### BRIEF DESCRIPTION OF THE DRAWINGS

Fig. 1 is a view illustrating metabolic pathways.
Fig. 2 is a block diagram of an optical measurement device.
Fig. 3 is a view illustrating the logic of estimating the activation balance between metabolic pathways.

### DESCRIPTION OF THE PREFERRED EMBODIMENTS

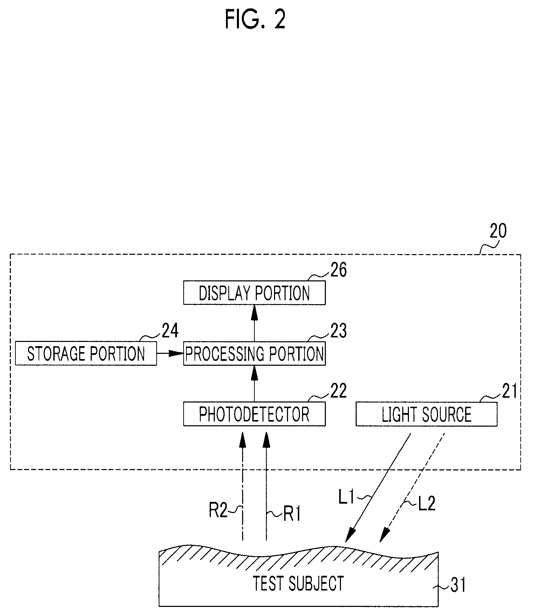

As shown in Fig. 2, an optical measurement device 20 includes at least one photodetector 22, and measures the amount of two or more biological substances contained in a test subject (biological body) 31 as a measurement subject based on the light absorption, light scattering, or light emission performed by the biological substances. The biological substances to be measured are biological substances whose amount varies in relation to the activation of at least one of the pathways of energy metabolism, nucleic acid metabolism, amino acid metabolism, or lipid metabolism. About a few (ten or less) biological substances are enough as the biological substances measured by the optical measurement device 20, and up to tens of (one hundred or less) biological substances are measured.

The optical measurement device 20 includes, for example, a light source 21, a processing portion 23, a storage portion 24, and a display portion 26. The light source 21 irradiates a test subject 31 with incidence light L1, which is for measuring light absorption or light scattering performed by the biological substances in the test subject 31, or with excitation light L2, which is for exciting a fluorescent substance or a phosphorescent substance in the test subject 31, according to the biological substances to be measured. For example, in case the light source 21 irradiates the test subject 31 with the incidence light L1, the photodetector 22 measures the amount of the corresponding predetermined biological substances by receiving reflected light R1 (or transmitted light) generated from the incidence light L1 that is absorbed (or scattered) by passing through the test subject 31 and attenuated. Furthermore, in case the light source 21 irradiates the test subject 31 with the excitation light L2, the photodetector 22 measures the amount of the corresponding predetermined biological substances by receiving fluorescence R2 that the test subject 31 emits by being irradiated with the excitation light L2.

The processing portion 23 estimates a degree of activation of at least two metabolic pathways among the aforementioned four metabolic pathways from the amount of the biological substances measured by the photodetector 22, and displays the results on the display portion 26. At this time, the amount of the substances measured may be taken as an estimated activation value of the metabolic pathways. In the storage portion 24, data which is necessary for the processing portion 23 to estimate a degree of activation (hereinafter, also referred to as an activation degree) of the metabolic pathways is stored in advance.

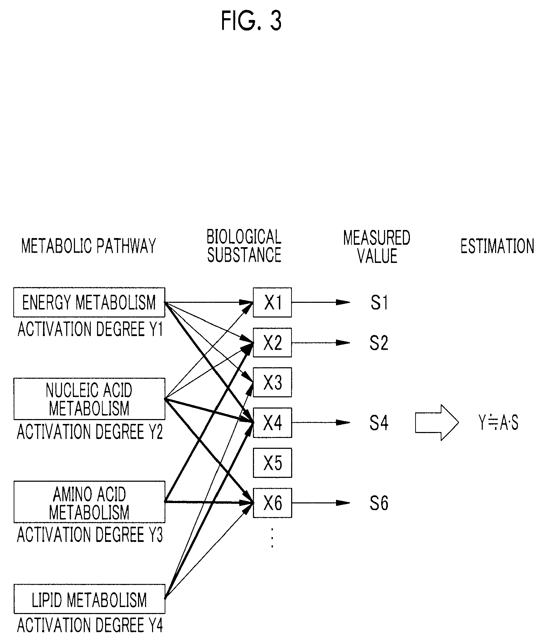

Based on the amount of a limited number of biological substances (for example, the amount of light received by the photodetector 22), the processing portion 23 estimates the activation degree of at least two metabolic pathways among the aforementioned four metabolic pathways. That is, the processing portion 23 functions as an activation degree calculation portion that calculates the activation degree of metabolic pathways. The processing portion 23 performs processing as shown in Fig. 3, for example. For instance, it is assumed that in the energy metabolism among four metabolic pathways of the energy metabolism, the nucleic acid metabolism, the amino acid metabolism, and the lipid metabolism, biological substances X1, X2, X3, and X4 are generated. Furthermore, the thickness of each arrow in Fig. 3 indicates the amount of the biological substances generated. In the energy metabolism, among the respective biological substances, particularly, the biological substance X4 is generated much. The same is true of other metabolic pathways. It is assumed that in the nucleic acid metabolism, the biological substances X1, X2, X4, and X6 are generated; in the amino acid metabolism, the biological substances X2 and X6 are generated; and in the lipid metabolism, the biological substances X3, X4, and X6 are generated.

In the optical measurement device 20, the amount of predetermined biological substances among the plurality of biological substances X1, X2, and the like is measured by the photodetector 22. In Fig. 3, the amount of the biological substances X1, X2, X4, and X6 is measured, and measurement values S1, S2, S4, and S6 are obtained. By calculating the weighted sum of the amount of the plurality of substances measured, the processing portion 23 estimates the activation of a first metabolic pathway. Furthermore, by using a different set of weight coefficients, the processing portion 23 estimates the activation of a second metabolic pathway. In the same manner, the processing portion 23 estimates the activation of third and fourth metabolic pathways. That is, by multiplying a vector S showing the amount of the biological substances measured (for example, a vector constituted with S1, S2, S4, and S6) by a weight coefficient matrix A constituted with weight coefficients for calculating the respective metabolic pathways, the processing portion 23 obtains, for example, a vector Y constituted with activation degrees Y1, Y2, Y3, and Y4 of the respective metabolic pathways. In this way, the activation degree of each of a plurality of pathways is estimated. The weight coefficient matrix A which is a set of weight coefficients is predetermined such that the activation of the plurality of metabolic pathways can be excellently estimated from the amount of the plurality of biological substances measured by the optical measurement device 20, and stored in the storage portion 24.

Two or more metabolic pathways selected from the aforementioned four metabolic pathways for the estimation of the activation thereof may be switchable. Furthermore, according to the combination of the metabolic pathways whose activation degree is to be estimated, the combination of the biological substances to be measured may be switched. In addition, according to the combination of the metabolic pathways whose activation degree is to be estimated, or according to the combination of the biological substances to be measured, a plurality of weight coefficient matrixes A may be prepared and switched such that the metabolic pathways whose activation is to be estimated are switched.

The test subject 31 which is to be measured by the optical measurement device 20 is a biological body. For example, the test subject 31 is a portion or the entirety of an animal including a human being, a sample cut off from an animal, or cells.

The optical measurement device 20 is, for example, a point measurement device (a contact type or a non-contact type), a biological image measurement device, or an endoscope (a soft endoscope or a hard endoscope).

The photodetector 22 is, for example, a photoelectric tube, a photomultiplier tube, a charge coupled device (CCD), or a complementary metal oxide semiconductor (CMOS) sensor.

The light source 21 is, for example, xenon, halogen, mercury, metal halide, a light emitting diode (LED), or a laser light source.

Among the biological substances to be measured, a light absorbing substance is, for example, oxidized hemoglobin, reduced hemoglobin, bilirubin, myoglobin, melanin, or lipofuscin.

Furthermore, among the biological substances to be measured, a light emitting substance is, for example, an autofluorescent substance which spontaneously exists in a biological body. Specifically, the light emitting substance is nicotinamide adenine dinucleotide (NADH), flavin adenine dinucleotide (FAD), collagen, porphyrin, elastin, melanin, lipofuscin, or the like.

The substance whose amount or activity varies in relation to the oxygen utilization by the metabolic pathway may be artificially caused to emit light. For example, a protein called prolyl hydroxylase domain-containing protein 2 (PHD2), whose amount varies in relation to the activation state of energy metabolism, may be expressed by being fused with a fluorescent protein by means of manipulating the gene of PHD2.

The biological substance that the optical measurement device 20 measures to estimate the activation of the metabolic pathways is preferably one of Acetyl-CoA carboxylase (ACC), ATP citrate lyase (ACLY), v-akt murine thymoma viral oncogene homolog (AKT), AMP-activated protein kinase (AMPK), carnitine palmitoyltransferase 1C (CPT1C), dihydrofolate reductase (DHFR), flavin adenine dinucleotide (FAD or FADH2), fatty acid synthase (FASN), Glucose-6-phosphate dehydrogenase (G6PD), Glutamate dehydrogenase (GDH), glutaminase (GLS), hypoxia-inducible factor-1 (HIF1), hexokinase 1 (HK1), isocitrate dehydrogenase (IDH), lactate dehydrogenase A (LDHA), monocarboxylate transporter 4 (MCT4), malic enzyme 1(ME1), monoglyceride lipase (MGLL), mammalian target of rapamycin (mTOR), v-myc avian myelocytomatosis viral oncogene homolog (MYC), nicotinamide adenine dinucleotide (NAD+ or NADH), NADH dehydrogenase, Nicotinamide adenine dinucleotide phosphate (NADP+ or NADPH), nicotinamide phosphoribosyl transferase (NAMPT), p53, pyruvate carboxylase (PC), Pyruvate dehydrogenase kinase (PDK), phosphofructokinase 2 (PFK2), phosphoglycerate mutase (PGAM), phosphoglycerate dehydrogenase (PHGDH), prolyl hydroxylase domain-containing protein 2 (PHD2), Phosphoinositide 3-kinase (PI3K), pyruvate kinase M2 (PKM2), Phosphatase and Tensin Homolog Deleted from Chromosome 10 (PTEN), ribonucleotide reductase (RNR), S-adenosylmethionine (SAM), SCO2 cytochrome c oxidase assembly protein (SCO2), serine/threonine kinase 11 (STK11), TP53-induced glycolysis and apoptosis regulator (TIGAR), transketolase-like 1 (TKTL1), and thymidylate synthetase (TYMS). Alternatively, the biological substance is preferably a combination of these.

The optical measurement device 20 may measure the amount of a biological substance based on the wavelength shift of Raman scattering light from the biological substance as a measurement subject.

The display portion 26 may display the estimated activation value of the metabolic pathway output from the processing portion 23, in the form of a numerical value, gray scale, color, or a graph.

In case the photodetector 22 is an image sensor such as CCD or CMOS, the display portion 26 may display the estimated activation value in the form of an image. In this case, the estimated activation value may be displayed in the form of gray scale, color, or the like for each pixel or for each of partial region (a region consisting of a plurality of pixels) of the test subject 31 as an observation subject. When a user designates a place in the image, the estimated activation value in the place may be displayed, for example, in the form of a numerical value, gray scale, color, or a graph that pops out. Furthermore, the images showing the respective estimated activation values may be displayed in a line or displayed by being temporally switched with each other. The images may be switched at a predetermined timing or switched based on the instruction from the user. Furthermore, a lookup table associating the plurality of estimated activation values with the gray scale or the color in the display may be preset such that display is performed based on the lookup table. For example, the lookup table may be a correspondence table in which the plurality of estimated activation values is associated with the intensity of channels of red, green, and blue of the display. The correspondence table is, for example, a linear correspondence table expressed in a matrix.

The display portion 26 may display the history of the parameter value in the form of a numerical value, gray scale, color, or a graph. Therefore, the display portion 26 may be provided with a storage portion which stores the estimated activation values of the past.

The optical measurement device 20 is preferable for, for example, diagnosis of cancer for the following reason. For instance, the optical measurement device 20 makes it possible to ascertain the metabolic change with the progress of cancer or the metabolic change after cancer therapy, and accordingly, highly accurate information helpful to determine a degree of malignancy, predict prognosis and drug responsiveness, select of a therapeutic method, and the like is obtained. Furthermore, the optical measurement device 20 can keep track of the activation balance of a plurality of metabolic pathways of the same measurement subject over time. Therefore, for example, in developing a drug, the optical measurement device 20 is useful for evaluating the drug efficacy or for obtaining a guideline regarding in what way the compound structure of the drug should be changed. In these cases, for improving the convenience of the user, the display portion 26 may display determination results showing whether or not the test subject 31 has cancer, the probability thereof, a degree of malignancy, the type of cancer, the prognosis prediction, the potency of drug efficacy, a suggested drug, and the like. These parameters useful for diagnosis or the like should be calculated by the processing portion 23 by using the activation degree of each of the metabolic pathways. In this case, the processing portion 23 functions as a parameter value calculation portion.

### Explanation of References

- 11:: cancer cell

- 20:: optical measurement device
- 21:: light source
- 22:: photodetector
- 23:: processing portion
- 24:: storage portion
- 26:: display portion

## Claims

1. An optical measurement device comprising:
a photodetector which measures the amount of a plurality of biological substances contained in a test subject by receiving light from the test subject; and
an activation degree calculation portion which calculates an activation degree of each of at least two metabolic pathways among energy metabolism, nucleic acid metabolism, amino acid metabolism, or lipid metabolism based on the amount of the plurality of biological substances detected by the photodetector.

2. The optical measurement device according to claim 1,
wherein the activation degree calculation portion calculates the activation degree by calculating the weighted sum of the amount of the plurality of biological substances.

3. The optical measurement device according to claim 2, further comprising:
a storage portion which stores in advance coefficients used for the activation degree calculation portion to calculate the weighted sum.

4. The optical measurement device according to claim 3,
wherein the storage portion stores in advance a plurality of sets of the coefficients according to a combination of the biological substances measured by the photodetector.

5. The optical measurement device according to claim 3 or 4,
wherein the storage portion stores in advance a plurality of the coefficients according to a combination of the metabolic pathways whose activation degree is to be calculated.

6. The optical measurement device according to any one of claims 1 to 5, further comprising:
a light source which irradiates the test subject with light according to the biological substances whose amount is to be measured by the photodetector.

7. The optical measurement device according to claim 6,
wherein the light source irradiates the test subject with light for measuring light absorption or light scattering performed by the biological substances, and
the photodetector measures the amount of the biological substances by receiving light reflected from or transmitted through the test subject.

8. The optical measurement device according to claim 6 or 7,
wherein the light source irradiates the test subject with excitation light for exciting a fluorescent substance or a phosphorescent substance in the test subject, and
the photodetector measures the amount of the fluorescent substance or the phosphorescent substance by receiving fluorescence or phosphorescence.

9. The optical measurement device according to any one of claims 1 to 8, further comprising:
a parameter calculation portion which calculates parameters helpful to determine a degree of malignancy of cancer, predict prognosis and drug responsiveness, or select a therapeutic method based on the activation degree of the metabolic pathways.

10. The optical measurement device according to any one of claims 1 to 9,
wherein the number of the biological substances of which the amount is measured by the photodetector is equal to or less than 100.

11. The optical measurement device according to claim 10,
wherein the number of the biological substances of which the amount is measured by the photodetector is equal to or less than 10.
